# FASCICULE DE BREVET EUROPEEN

(11) **EP 2 632 558 B1**
(45) Date de publication et mention de la délivrance du brevet: **30.05.2018**
(21) Numéro de dépôt: 11772939.2
(22) Date de dépôt: 17.10.2011
(51) Int. Cl.: B01D 3/00, C07C 67/08, C07C 69/68, C07C 67/54

(54) **PROCÉDÉ DE PRODUCTION D'UN ESTER LACTIQUE À PARTIR D'UN JUS DE FERMENTATION CONTENANT DU LACTATE D'AMMONIUM**
VERFAHREN ZUR HERSTELLUNG EINES MILCHSÄUREESTERS AUS EINER FERMENTATIONSBRÜHE ENTHALTEND AMMONIUMLACTAT
PROCESS FOR PRODUCING A LACTIC ESTER FROM A FERMENTATION LIQUOR CONTAINING AMMONIUM LACTATE

(30) Priorité: 28.10.2010 BE 201000639
(43) Date de publication de la demande: 04.09.2013
(73) Titulaire: Galactic S.A., 7760 Escanaffles (BE)
(72) Inventeur: BERNARD, Aurélie, B-7760 Escanaffles (BE); MARIAGE, Pierre-Antoine, B-7760 Escanaffles (BE); BOGAERT, Jean-Christophe, B-7760 Escanaffles (BE)
(74) Mandataire: Pronovem
(86) Numéro de dépôt international: PCT/EP2011/068066
(87) Numéro de publication internationale: WO 2012/055717

(56) Documents cités:
- EP-A2- 0 614 983
- WO-A1-2007/013259
- US-A1- 2003 029 711
- KUMAR, RAKESH; MAHAJANI, SANJAY M.: "Esterification of Lactic Acid with n-Butanol by Reactive Distillation", INDUSTRIAL & ENGINEERING CHEMISTRY RESEARCH, vol. 46, no. 21, 7 septembre 2007 (2007-09-07), pages 6873-6882, XP002635272, ISSN: 0888-5885, DOI: 10.1021/ie061274j

## Description

### Domaine de l'invention

La présente invention se rapporte à un procédé industriel de production d'un ester lactique sans production simultanée de gypse. En particulier, la présente invention se rapporte à un procédé industriel de production d'un ester lactique contenant au total au moins sept atomes de carbone, à partir d'un jus de fermentation contenant du lactate d'ammonium afin d'éviter la production inhérente de gypse, avec un rendement élevé et selon lequel on parvient à limiter au maximum la perte d'ester lactique sous forme de lactamide.

### Etat de l'art

On connaît très bien la production d'acide lactique par le procédé classique de fermentation qui consiste en la transformation par un microorganisme d'une substance carbonée (glucose, sucrose, amidon, cellulose, fructose,...) en présence de carbonate de calcium. La solution obtenue est alors traitée par un acide afin de libérer l'acide lactique sous forme protonée mais cette étape d'acidification implique la production de sulfate de calcium (aussi dénommé gypse). Or, il est bien connu de l'homme de l'art, que la formation de gypse implique que le précipité doit alors être filtré tandis que l'acide lactique sera purifié et concentré par des techniques telles que les résines échangeuses d'ions, la décoloration sur charbon, la nanofiltration, l'évaporation, la distillation, l'extraction liquide/liquide.

De plus, à l'échelle industrielle, la valorisation du gypse devient problématique, en effet, les quantités produites sont importantes et le gypse produit peut difficilement être valorisé. Il doit être éliminé comme un déchet, entraînant des coûts non négligeables et un impact sur l'environnement. La production d'acide lactique sans gypse est donc d'un grand intérêt.

Afin de remédier à cet inconvénient important, on a déjà proposé, comme dans le brevet US 2565487 ou le brevet WO 2007013259, de produire des esters d'acide lactique à partir de sels d'ammonium et plus particulièrement le lactate d'ammonium, et d'alcool. L'ester produit peut, dans une seconde étape être hydrolysé en acide lactique. Ces procédés n'étant pas réalisés en continu.
Il est également connu que lors de la fermentation, l'acide lactique produit par les micro-organismes peut être neutralisé avec de l'ammoniaque. Le lactate d'ammonium ainsi formé peut être estérifié à l'aide d'un alcool pour former l'ester lactique correspondant.
Or, il est bien connu également que la voie de production passant par les sels d'ammonium présente aussi de nombreux inconvénients, qui rendent notamment difficile la transposition à l'échelle industrielle d'un procédé utilisant cette voie.

En effet, lors de la concentration du jus de fermentation ainsi que pendant l'étape d'estérification, il a été observé la formation d'un sous-produit indésirable, le lactamide.

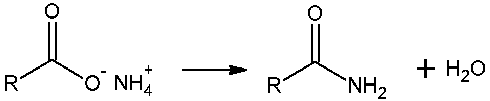

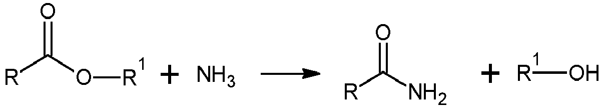

La formation de lactamide lors de la conversion du lactate d'ammonium en ester lactique diminue la sélectivité de la réaction, entraine une perte en rendement et nécessite une séparation et une élimination supplémentaire.
Comme expliqué dans le brevet WO 2006/069113, pour éviter la formation de lactamide, il est nécessaire de travailler avec un large excès molaire d'alcool compris entre 20 et 100 moles d'alcool par mole de lactate d'ammonium. La purification de l'ester formé par distillation de l'alcool en large excès nécessite dés lors un apport énergétique important incompatible avec une utilisation industrielle rentable du procédé.

Par ailleurs, lors de l'utilisation d'un jus de fermentation lactique sous forme de lactate d'ammonium non purifié, les étapes de concentration et d'estérification sont rendues difficiles par la précipitation de sous-produits de la fermentation (résidus de sucre,...). Cette précipitation entraîne un faible rendement d'estérification.

Non seulement, l'état de la technique permet de bien connaître les problèmes relatifs à la présence de lactamide, mais en plus l'état de la technique mentionne également le problème de la racémisation des esters obtenus à partir d'un jus de fermentation lactique sous forme de lactate d'ammonium, comme décrit par M.ALAS dans le brevet EP 517571B1, où l'on enseigne la possibilité de produire des esters lactiques avec un taux de racémisation supérieur à 2%, ce qui est bien connu pour être néfaste pour l'ester obtenu.
L'article « Esterification of lactic acid with n-butanol by reactive distillation » (Ind. Eng. Chem. Res. 2007, 46, 6873-6882*)* mentionne la production d'ester lactique (hydrolysable en acide lactique) par estérification de l'acide lactique et non au départ d'un jus de fermentation, il ne résout donc pas les problèmes liés aux lactamides et ne propose pas un procédé complet de la fermentation jusqu'à l'acide lactique.

Il existe donc un besoin pour un procédé industriel de production d'ester lactique sans production conjointe de gypse, et qui permette de remédier aux inconvénients mentionnés ci-dessus.

Un des objets de la présente invention est un procédé qui permet de remédier à ces problèmes, au départ d'un jus de fermentation sous forme de lactate d'ammonium.
Un autre objet de l'invention est un procédé permettant d'obtenir un acide lactique sans formation de gypse, avec un rendement élevé, généralement > 95% et dont la perte sous forme de lactamide n'excède pas 5% lors de l'étape d'estérification.
Encore un objet de l'invention est un procédé qui permet de limiter la racémisation à un niveau inférieur à 2%.

### Brève description de l'invention

La Demanderesse a maintenant trouvé d'une manière inattendue que l'on pouvait produire un ester lactique avec un nombre total de carbone supérieur ou égal à 7 à partir d'un flux de lactate d'ammonium avec un rendement supérieur à 95 %, en soumettant à une distillation réactive un courant de lactate d'ammonium, provenant d'un jus de fermentation purifié et concentré, sous forme liquide, conjointement avec un courant d'un alcool aliphatique de 4 à 8 atomes de carbone, sous forme vapeur, en récupérant en pied de colonne un courant liquide de l'ester lactique correspondant et en tête de colonne un mélange gazeux comprenant l'excès d'alcool, l'ammoniaque et l'eau.

### Description détaillée de l'invention

La société demanderesse a trouvé un procédé de production d'un ester lactique à partir d'un flux de lactate d'ammonium, n'impliquant pas de production de gypse et permettant de remédier aux inconvénients mentionnés ci-dessus concernant la production par la voie des sels d'ammonium.
Le procédé de l'invention comprend successivement les étapes suivantes : on réalise tout d'abord une pré-purification d'un jus de fermentation sous forme de lactate d'ammonium; ce jus de fermentation pré-purifié est ensuite soumis à une concentration et ensuite à une distillation réactive en présence d'un courant gazeux d'un alcool aliphatique contenant 4 à 8 atomes de carbone afin de réaliser l'estérification du lactate d'ammonium; ces deux dernières opérations étant réalisées dans un seul et même appareillage constitué de 3 étages; l'ester ainsi produit est récupéré et ensuite purifié par distillation.

### 1. La pré-purification du jus de fermentation

La demanderesse a observé de manière surprenante que les différentes étapes de la pré-purification permettaient non seulement une diminution du sous-produit lactamide mais également du taux de racémisation.
La première étape de la pré-purification consiste en une élimination de la biomasse par toutes techniques connues de l'homme de l'art telles que, de manière non-limitative, la filtration sur pré-couche, la filtration membranaire, la décantation ou la centrifugation. Cette étape est suivie d'une décationisation du jus de fermentation sur une résine échangeuse d'ions préalablement conditionnée sous forme ammoniacale. Le jus de fermentation débarrassé de ses cations divalents est alors traité par nano-filtration.

### 2. La concentration du jus de fermentation pré-purifié

Le jus de fermentation ainsi purifié est ensuite concentré jusqu'à l'obtention d'une concentration comprise entre 50 et 80%.

Une version préférée de la présente invention consiste à exploiter les techniques d'évaporation sur couche mince pour cette étape de concentration qui permettent à nouveau de limiter la formation de lactamide. Dans ces conditions, il n'a pas non plus été observé de racémisation durant cette étape.

### 3. L'estérification du lactate d'ammonium

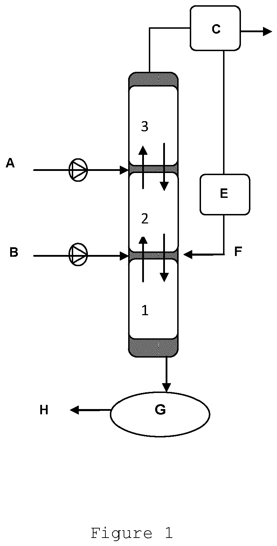

L'estérification du lactate d'ammonium concentré est réalisée à contre-courant d'un alcool contenant au moins 4 atomes de carbone, sous forme gazeuse dans un appareillage (figure 1) permettant un bon échange liquide/gaz. On a constaté que des temps de séjour inférieurs à 2h conviennent parfaitement et que d'excellents résultats peuvent même être obtenus avec des temps de séjour inférieurs à 30 min. L'appareillage utilisé est constitué d'une colonne de distillation réactive à 3 étages de sorte que :
a) Les vapeurs sortent de l'étage 1 (nombre de plateaux théoriques = 20) par la tête de l'étage 1 dans le pied de l'étage 2 et les vapeurs sortent de l'étage 2 (nombre de plateaux théoriques = 15) par la tête de l'étage 2 dans le pied de l'étage 3 (nombre de plateaux théoriques = 10)
b) Les liquides de l'étage 2 sortent par le pied de l'étage 2 dans la tête de l'étage 1 et les liquides de l'étage 3 sortent par le pied de l'étage 3 dans la tête de l'étage 2.
c) Le lactate d'ammonium d'une concentration comprise entre 10 et 80 % est introduit en tête de l'étage 2
d) De l'alcool avec C ≥ 4 sous forme de vapeur provenant de la tête de l'étage 1, est introduit en pied de l'étage 2
e) L'ester lactique formé est récupéré en pied de l'étage 1 avec une teneur en lactamide inférieure à 5%
f) Le mélange gazeux alcool (C ≥ 4), eau et ammoniaque est récupéré en tête de l'étage 3
g) L'alcool (C ≥ 4) est séparé de l'eau et de l'ammoniaque par décantation et est réintroduit en pied de l'étage 2

L'alcool est chauffé à sa température d'ébullition (la température et la pression étant dépendantes de l'alcool utilisé) et introduit dans la colonne en pied de l'étage 2 via la canalisation B de la figure 1, sous forme de gaz. La solution précédemment pré-purifiée et concentrée de lactate d'ammonium, dans lequel un catalyseur, préférentiellement mais non limitativement l'acide para-toluène sulfonique (APTS), est éventuellement ajouté de manière à accélérer la réaction, est introduit en tête de l'étage 2 via la canalisation A de la figure 1. Les vapeurs ascendantes d'alcool croisent donc à contre-courant le flux liquide descendant de lactate d'ammonium. L'eau formée lors de l'estérification est entrainée par les vapeurs d'alcool ascendantes et récupérée sur le condenseur C en tête de l'étage 3 repris sur la figure 1. L'ammoniac libéré lors de la réaction est également emporté et se retrouve dans cette même phase aqueuse.

L'excès d'alcool aussi récupéré sur le condenseur C est alors séparé de la phase aqueuse contenant l'ammoniac en,C par décantation, puis chauffé et gazéifié dans un échangeur E et réintroduit en F en pied de l'étage 2. Le rapport molaire alcool/lactate d'ammonium est compris entre 4 :1 et 2 :1, préférentiellement 3 :1. Dans le cas où l'alcool est le butanol, le procédé peut être conduit à une température de 120°C et à pression atmosphérique.

Le dimensionnement des étages de la colonne peut être facilement effectué par l'homme de l'art mais, à titre d'information non-limitative, on peut considérer que les étages 1, 2 et 3 sont constitués de garnissage structuré ou non et contiennent respectivement 20, 15 et 10 plateaux théoriques. Dans le cadre de l'invention, ces étages peuvent en outre aussi être constitués de plateaux à cloches ou de tout autre profile de colonne connu de l'homme de l'art pour favoriser les échanges liquides-vapeurs.

Ce procédé permet la récupération, via la canalisation H en sortie du rebouilleur G de la figure 1, d'un ester lactique avec un rendement supérieur à 95% dont la perte en lactamide est inférieure à 5% et la racémisation inférieure à 2%.

### 4. Purification de l'ester lactique produit

L'ester lactique ainsi formé peut si nécessaire être purifié par toutes techniques connues de l'homme de l'art, pour autant que cette étape n'amène pas de nouvelle racémisation. Le résidu de distillation peut être récupéré et recyclé à l'étape d'estérification. L'ester lactique purifié peut le cas échéant être hydrolysé en acide lactique. Il est dans ce cas possible d'atteindre des grades de hautes qualités répondant aux critères du marché.

D'autres détails et particularités de l'invention, donnés ci-après à titre d'exemples non-limitatifs, ressortent de la description comme quelques formes possibles de sa réalisation.

### Exemple 1 : Estérification du lactate d'ammonium dans l'appareillage de l'invention

Le jus de fermentation a été filtré sur pré-couche de dicalite pour éliminer la biomasse. Le filtrat a ensuite été traité sur résines cationiques LEWATIT 2528 pré-conditionnée sous forme ammoniaque afin de ne pas dépasser 20 ppm en cations divalents. Le débit était fixé à 2 BV/h (un BV ou « bed volume » correspond au volume occupé par le lit de résine dans la colonne de traitement).

Ce jus de fermentation est ensuite traité par nano-filtration, avec un débit d'alimentation compris entre 500 et 600 ml/h, sur un pilote de nanofiltration osmonics SEPACF2 avec une membrane GEWATER (DL).
Enfin, le jus de fermentation est concentré, dans un appareil à couche mince, à une pression comprise entre 90 et 120 mbar et une température comprise entre 100 et 105°C avec un débit d'alimentation de 6 1/h, jusqu'à obtenir une concentration de 60%.
Une colonne correspondant au schéma de la figure 1 est alimentée par du butanol sous forme de vapeur à 120°C à pression atmosphérique, en tête de l'étage 1 (Figure 1, position B) à raison de 550 g/h. L'étage 1 est consitué de garnissage structuré et contient 20 plateaux théoriques. Le rebouilleur G en pied de l'étage 1 est constitué d'un échangeur à plaques chauffé à 140°C. Le lactate d'ammonium pré-purifié et concentré à 60% est mélangé à 1% d'APTS pour être introduit en tête de l'étage 2 (Figure 1, position A) à raison de 1204 g/h. L'étage 2 contient le même garnissage structuré que l'étage 1 pour un nombre de plateaux théoriques de 15. L'eau formée et le butanol sont entrainés en tête de l'étage 3 (Figure 1, position C). L'étage 3 est lui aussi composé de garnissage structuré et contient 10 plateaux théoriques. La phase aqueuse contenant l'ammoniac recueilli sur le condenseur en position C est alors séparée du butanol par décantation, Le butanol recueilli après décantation est lui chauffé à ébullition (Figure 1, position E) dans un échangeur de chaleur et retourné en tête de l'étage 1 (Figure 1, position F), pour assurer l'équilibre, à une vitesse d'alimentation de 1500 g/h. Le lactate de butyle est récupéré en pied de l'étage 1 ((Figure 1, position H). Un suivi de la réaction en fonction du temps a été réalisé, les résultats sont repris dans le tableau 1.

**Tableau 1 : caractéristiques du lactate de butyle produit en fonction du temps**

| **Temps (h)** | **Lactate de butyle sorti (g/h)** | **Conc. en lactate de butyle**^{(**a**)} **(%)** | **Conc. en lactamide (%)** | **Conc. en eau**^{(**b**)} **(%)** | **Racémisation (%)** | **Perte en lactamide (%)** | **Rendement (%)** |
|---|---|---|---|---|---|---|---|
| 1 | 980,3 | 95 | 2 | 0,19 | 0 | 3,3 | 96 |
| 2 | 988,7 | 96 | 2 | 0,17 | 0 | 3,3 | 96 |
| 4 | 979,4 | 96.7 | 1,5 | 0,18 | 0 | 2,4 | 97 |
| 8 | 997,8 | 97 | 1 | 0,18 | 0 | 1,7 | 98 |
| 10 | 997 | 97 | 1,5 | 0,17 | 0 | 2,5 | 97 |
| 12 | 985,9 | 97.5 | 1 | 0,19 | 0 | 1.7 | 98 |

| | | | | | | | |
|---|---|---|---|---|---|---|---|
| (a) déterminé par mesure Chromatographie en phase gazeuse (b) déterminé par mesure Karl Fisher | | | | | | | |

Comme le montre le tableau 1, l'appareillage permet de produire du lactate de butyl à partir de lactate d'ammonium de manière stable (sur 12h) avec un rendement supérieur à 95%, une perte sous forme de lactamide inférieure à 5% et une racémisation inférieure à 2%.

### Exemple 2 : Estérification d'un jus de fermentation pré-purifié sous forme lactate d'ammonium en lactate de butyle avec et sans utilisation d'une colonne

222 g de butanol sont placés dans un ballon et chauffés à 120°C à pression atmosphérique, 1% d'APTS (acide para-toluène sulfonique) est ajouté. Lorsque le butanol distille, 178,3 g d'un jus de fermentation pré-purifié sous forme de lactate d'ammonium 60% sont ajoutés goutte à goutte dans le ballon. La réaction est poursuivie jusqu'à l'obtention d'une acidité résiduelle nulle. En fin de synthèse, le produit de réaction est analysé, les résultats sont repris dans le tableau 2.

**Tableau 2 : caractéristiques du lactate de butyle produit**

| **Concentration en lactate de butyle**^{(**a**)} **(%)** | **Rendement d'estérification (%)** | **Perte en lactamide (%)** | **Concentration en eau^{(b)} (%)** | **Racémisation (%)** |
|---|---|---|---|---|
| 40,6 | 77 | 10 | 0,9 | 2 |

| | | | | |
|---|---|---|---|---|
| (c) déterminé par mesure Chromatographie en phase gazeuse (d) déterminé par mesure Karl Fisher | | | | |

On peut remarquer que la conversion du lactate d'ammonium en lactate de butyle n'est pas très élevée et que par contre la perte en lactamide est importante.

Un nouvel essai est réalisé en surmontant le ballon d'une colonne contenant un garnissage structuré, afin d'effectuer l'estérification à contre courant de l'alcool sous forme gazeuse.
222 g de butanol sont placés dans un ballon surmonté de cette colonne et chauffés à 120°C à pression atmosphérique, 1% d'APTS est ajouté. Lorsque le butanol distille en tête de colonne, 178,3 g d'un jus de fermentation pré-purifié sous forme de lactate d'ammonium 60% sont ajoutés goutte à goutte en tête de colonne. La réaction est poursuivie jusqu'à l'obtention d'une acidité résiduelle nulle. En fin de synthèse, le produit de réaction est analysé, les résultats sont repris dans le tableau 3.

**Tableau 3 : caractéristiques du lactate de butyle produit**

| **Concentration en lactate de butyle (%)** | **Rendement d'estérification (%)** | **Perte en lactamide (%)** | **Concentration en eau (%)** | **Racémisation (%)** |
|---|---|---|---|---|
| 50 | 96,2 | 4 | 0,18 | 0 |

Le fait d'utiliser une colonne permet non seulement d'augmenter la conversion du lactate d'ammonium en lactate de butyle mais également de diminuer la perte en lactamide ainsi que la racémisation.
Le lactate de butyle produit est ensuite purifié par distillation, les caractéristiques sont reprises dans le tableau 4.

**Tableau 4 : Caractéristiques du lactate de butyle distillé**

| **Concentration en lactate de butyle (%)** | **Rendement de distillation (%)** | **Perte en lactamides(%)** | **Concentration en eau (%)** | **Racémisation (%)** |
|---|---|---|---|---|
| 98 | 77 | 2 | 0,08 | 0 |

### Exemple 3 : Concentration du jus de fermentation avec et sans pré-purification

Une fermentation a été réalisée avec une neutralisation à l'ammoniaque.
300 g de jus de fermentation sont directement concentrés en ballon. Les caractéristiques du jus de fermentation concentré sous forme lactate d'ammonium sont reprises dans le tableau 5.

**Tableau 5 : caractéristiques du jus de fermentation après concentration**

| **Acide lactique**^{(**a**)} **(g/l)** | **Lactamide**^{(**b**)} **(ppm)** | **Rendement (%)** | **Racémisation**^{(**c**)} **(%)** |
|---|---|---|---|
| 440 | 136 000 | 77 | 5 |

| | | | |
|---|---|---|---|
| (a) déterminé par titrage (b) déterminé par mesure HPLC (c) déterminé par dosage enzymatique | | | |

On observe une importante formation du sous-produit lactamide lors de l'étape de concentration.

Une nouvelle fermentation a été réalisée avec une neutralisation à l'ammoniaque, cette fois, le jus de fermentation a été filtré sur pré-couche de dicalite pour éliminer la biomasse. Le filtrat a ensuite été traité sur résines cationiques LEWATIT 2528 pré-conditionnée sous forme ammoniaque afin de ne pas dépasser 20 ppm en cations divalents. Le débit était fixé à 2 BV/h (un BV correspond au volume occupé par le lit de résine dans la colonne de traitement).

Ce jus de fermentation est ensuite nano-filtré, avec un débit d'alimentation compris entre 500 et 600 ml/h, sur un pilote de nanofiltration osmonics SEPACF2 avec une membrane GEWATER (DL).

Enfin, le jus de fermentation est concentré dans un appareil à couche mince à une pression comprise entre 90 et 120 mbar et une température comprise entre 100 et 105°C avec un débit d'alimentation de 6 l/h, jusqu'à obtenir une concentration de 60%.
Les caractéristiques de ce jus de fermentation sous forme lactate d'ammonium sont reprises dans le tableau 6.

**Tableau 6 : caractéristiques du jus de fermentation pré-purifié après concentration**

| **Total LA**^{(**a**)} **(g/l)** | **Lactamide (ppm)** | **Rendement (%)** | **Racémisation (%)** |
|---|---|---|---|
| 540 | 3400 | 99,4% | 1 |

| | | | |
|---|---|---|---|
| (a) Lactate d'ammonium + acide lactique | | | |

On peut remarquer que la pré-purification sur résine suivie d'une nanofiltration et de la concentration sur couche mince, permet de diminuer considérablement la formation de lactamide et la racémisation lors de la concentration.

### Exemple 4 : Estérification en lactate de 2-éthylhexyle d'un jus de fermentation sous forme lactate d'ammonium

390 g de 2-éthylhexanol sont placés dans un ballon surmonté d'une colonne.et chauffés à 130°C à une pression de 100 mbar, 0,1% d'APTS sont ajoutés. Lorsque le 2-éthylhexanol distille, 178,3 g de lactate d'ammonium pré-purifié et concentré à 60% sont ajoutés goutte à goutte en tête de colonne. La réaction est poursuivie jusqu'à l'obtention d'une acidité résiduelle nulle. En fin de synthèse le produit de réaction est analysé, les résultats sont repris dans le tableau 7.

**Tableau 7 : Caractéristiques du lactate de 2-éthylhexyle produit**

| **Concentration en lactate de 2-éthylhexyle^{(a)} (%)** | **Rendement d'estérification (%)** | **Perte en lactamide (%)** | **Concentration en eau (%)** | **Racémisation (%)** |
|---|---|---|---|---|
| 67 | 97 | 2 | 0,1 | 0,3 |

| | | | | |
|---|---|---|---|---|
| (a) déterminé par Chromatographie en phase gazeuse | | | | |

Le lactate de 2-éthylhexyle produit est ensuite purifié par distillation, les caractéristiques sont reprises dans le tableau 8.

**Tableau 8 : Caractéristiques du lactate de 2-éthylhexyle distillé**

| **Concentration en lactate de 2-éthylhexyle (%)** | **Rendement de distillation (%)** | **Concentration en lactamide (%)** | **Concentration en eau (%)** | **Racémisation (%)** |
|---|---|---|---|---|
| 98 | 95 | 0,06 | 0,05 | 0,3 |

On peut remarquer la très faible concentration en lactamide et le taux de racémisation faible également.

### Exemple 5 : Hydrolyse du lactate de butyle produit à l'exemple 2 en acide lactique de haute qualité

292 g de lactate de butyle, 288 g d'eau déminéralisée et 1% d'APTS sont placés dans un ballon et chauffés à 105°C à pression atmosphérique. La réaction est poursuivie pendant 8h. En fin d'hydrolyse, le produit de réaction est analysé, les résultats sont repris dans le tableau 9.

**Tableau 9 : Caractéristiques du lactate de butyle hydrolysé**

| **Concentration en lactate de butyle (%)** | **Rendement d'hydrolyse (%)** | **Acide lactique (g/l)** | **Concentration en eau (%)** | **Racémisation (%)** |
|---|---|---|---|---|
| 0,6 | 99 | 510 | 0,08 | 0 |

99 % du lactate de butyle ont été hydrolysés en acide lactique sans provoquer de racémisation de cet acide lactique.
L'acide lactique ainsi obtenu est concentré afin de répondre au critère de qualité du marché. Les résultats sont repris dans le tableau 10.

**Tableau 10 : Caractéristiques de l'acide lactique concentré**

| **Acide lactique (%)** | **Coloration (Hazen)** | **Racémisation (%)** |
|---|---|---|
| 83,2 | 55 | 0 |

## Revendications

1. Procédé de production d'un ester lactique contenant au total au moins 7 atomes de carbone à partir d'un flux de lactate d'ammonium, **caractérisé en ce que** l'on réalise une distillation réactive en colonne d'un courant de lactate d'ammonium sous forme liquide, pré-purifié par:
a) élimination de la biomasse ;
b) décationisation du jus de fermentation sur une résine échangeuse d'ions préalablement conditionnée sous forme amoniacale ;
c)nanofiltration du jus décationnisé,
et ledit lactate pré-purifié étant également concentré, simultanément à un courant d'un alcool aliphatique contenant de 4 à 8 atomes de carbone, sous forme vapeur, et que l'on récupère en pied de colonne un courant liquide de l'ester lactique correspondant et en tête de colonne un mélange gazeux comprenant l'alcool aliphatique, l'ammoniac et l'eau.

2. Procédé selon la revendication 1, **caractérisé en ce que** l'on concentre le courant de lactate d'ammonium prépurifié jusqu'à un niveau de concentration compris entre 50 et 80%.

3. Procédé selon la revendication 2, **caractérisé en ce que** l'on réalise la concentration par évaporation sur couche mince.

4. Procédé selon les revendications 1 à 3, **caractérisé en ce que**, lors de la distillation réactive, le rapport molaire entre l'alcool et le lactate d'ammonium est compris entre 2 :1 et 4 :1 .

5. Procédé selon les revendications 1 à 4, **caractérisé en ce que** l'alcool aliphatique que l'on utilise pour la distillation réactive est choisi parmi le butanol, l'isoamylalcool et le 2-éthylhexanol.

6. Procédé selon les revendications 1 à 3, **caractérisé en ce que** le résidu de distillation de l'ester lactique produit est récupéré et recyclé à l'étape d'estérification.

7. Procédé selon les revendications 1 à 6, **caractérisé en ce que** l'ester lactique contenant au-moins 7 atomes de carbone est hydrolysé en acide lactique.

8. Procédé selon la revendication 7 **caractérisé en ce que** l'alcool issu de l'hydrolyse de l'ester lactique contenant au-moins 7 atomes de carbone est recyclé à l'étape d'estérification réactive.

## Patentansprüche

1. Verfahren zur Herstellung eines Milchsäureesters, enthaltend insgesamt mindestens 7 Kohlenstoffatome, aus einem Ammoniumlaktatfluss, **dadurch gekennzeichnet, dass** eine reaktive Destillation in einer Säule eines Ammoniumlaktatstroms in flüssiger Form durchgeführt wird, vorgereinigt durch:
a) Entfernen der Biomasse,
b) Dekationisieren der Fermentationsbrühe auf einem zuvor in ammoniakalischer Form konditionierten Ionentauscherharz,
c) Nanofiltrieren der dekationisierten Brühe,
wobei das vorgereinigte Laktat ebenfalls gleichzeitig mit einem Strom eines aliphatischen Alkohols, enthaltend 4 bis 8 Kohlenstoffatome, in Dampfform konzentriert wird, und dass am Säulenfuß ein entsprechender flüssiger Milchsäureesterstrom und am Säulenkopf ein Gasgemisch, umfassend den aliphatischen Alkohol, Ammoniak und Wasser, zurückgewonnen wird.

2. Verfahren nach Anspruch 1, **dadurch gekennzeichnet, dass** der vorgereinigte Ammoniumlaktatstrom bis auf einen Konzentrationsgrad zwischen 50 und 80 % inklusive konzentriert wird.

3. Verfahren nach Anspruch 2, **dadurch gekennzeichnet, dass** die Konzentration durch Verdampfen auf Dünnschicht durchgeführt wird.

4. Verfahren nach den Ansprüchen 1 bis 3, **dadurch gekennzeichnet, dass** bei der reaktiven Destillation das molare Verhältnis zwischen dem Alkohol und dem Ammoniumlaktat zwischen 2 : 1 und 4 :1 inklusive beträgt.

5. Verfahren nach den Ansprüchen 1 bis 4, **dadurch gekennzeichnet, dass** der aliphatische Alkohol, der für die reaktive Destillation verwendet wird, aus dem Butanol, dem Isoamylalkohol und dem 2-Ethylhexanol ausgewählt ist.

6. Verfahren nach den Ansprüchen 1 bis 3, **dadurch gekennzeichnet, dass** der Destillationsrest des hergestellten Milchsäureesters beim Veresterungsschritt zurückgewonnen und recycelt wird.

7. Verfahren nach den Ansprüchen 1 bis 6, **dadurch gekennzeichnet, dass** der Milchsäureester, der mindestens 7 Kohlenstoffatome enthält, in Milchsäure hydrolysiert wird.

8. Verfahren nach Anspruch 7, **dadurch gekennzeichnet, dass** der Alkohol im Ergebnis der Hydrolyse des Milchsäureesters, der mindestens 7 Kohlenstoffatome enthält, beim Schritt der reaktiven Veresterung recycelt wird.

## Claims

1. Method for producing a lactic ester containing a total of at least 7 carbon atoms from a flow of ammonium lactate, **characterized in that** reactive distillation is performed, on a column, on a flow of ammonium lactate in liquid form, pre-purified by:
a) removing the biomass;
b) decationizing the fermentation liquor on an ion exchange resin previously conditioned in ammoniacal form;
c) nanofiltering the decationized liquor,
and said pre-purified lactate also being concentrated, simultaneously with a flow of an aliphatic alcohol having 4 to 8 carbon atoms, in vapour form, a corresponding liquid flow of lactic ester being recovered at the foot of the column and a gaseous mixture comprising the aliphatic alcohol, ammonia and water being recovered at the head of the column.

2. The method according to claim 1, **characterized in that** the flow of pre-purified ammonium lactate is concentrated to a concentration level of between 50 and 80 %.

3. The method according to claim 2, **characterized in that** concentration is conducted via thin layer evaporation.

4. The method according to claims 1 to 3 **characterized in that**, at the time of reactive distillation, the molar ratio between the alcohol and ammonium lactate is between 2:1 and 4:1.

5. The method according to claims 1 to 4, **characterized in that** the aliphatic alcohol used for reactive distillation is selected from among butanol, isoamyl alcohol and 2-ethylhexanol.

6. The method according to claims 1 to 3, **characterized in that** the residue produced from lactic ester distillation is recovered and recycled at the esterification step.

7. The method according to claims 1 to 6, **characterized in that** the lactic ester containing at least 7 carbon atoms is hydrolysed to lactic acid.

8. The method according to claim 7, **characterized in that** the alcohol derived from hydrolysis of the lactic ester containing at least 7 carbon atoms is recycled at the reactive esterification step.
